# EUROPÄISCHE PATENTSCHRIFT

(11) **EP 0 993 446 B1**
(45) Veröffentlichungstag und Bekanntmachung des Hinweises auf die Patenterteilung: **03.11.2004**
(21) Anmeldenummer: 98939502.5
(22) Anmeldetag: 15.06.1998
(51) Int. Cl.: C07D 211/90

(54) **VERFAHREN ZUR HERSTELLUNG VON NIFEDIPIN**
PROCESS FOR PREPARING NIFEDIPINE
PROCEDE DE PREPARATION DE NIFEDIPINE

(30) Priorität: 27.06.1997 DE 19727350
(43) Veröffentlichungstag der Anmeldung: 19.04.2000
(73) Patentinhaber: Bayer HealthCare AG, 51368 Leverkusen (DE)
(72) Erfinder: BERWE, Mathias, D-45549 Sprockhövel (DE); DIEHL, Herbert, D-51373 Leverkusen (DE); RITTNER, Karl, D-51519 Odenthal (DE); WAHL, Karl-Heinz, D-51519 Odenthal (DE); WIRGES, Hans-Peter, D-47800 Krefeld (DE)
(86) Internationale Anmeldenummer: PCT/EP1998/003591
(87) Internationale Veröffentlichungsnummer: WO 1999/000369

(56) Entgegenhaltungen:
- EP-A- 0 124 742
- DD-A- 290 878
- DE-A- 4 423 445
- ES-A- 8 600 745
- DATABASE WPI Section Ch, Week 9651 Derwent Publications Ltd., London, GB; Class B03, AN 96-517020 XP002084861 & RU 2 057 122 C (KRISTALL SCI PRODN ASSOC), 27. März 1996
- WATANABE ET AL.: "An efficient procedure for the Hantzsch dihydropyridine synthesis" SYNTHESIS,1983, Seite 761 XP002084578

## Beschreibung

Die Erfindung betrifft ein neues Verfahren zur Herstellung von Nifedipin.

Die Verbindung 1,4-Dihydro-2,6-dimethyl-4-(2-nitrophenyl}-3,5-pyridindicarbonsäuredimethylester der Formel 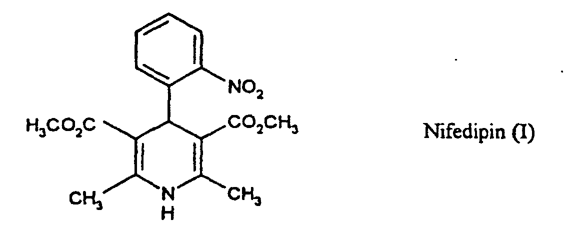 ist als Arzneimittelwirkstoff Nifedipin bekannt (US-A-3 485 847).

Nifedipin ist ein anerkannter Arzneimittelwirkstoff, der weltweit in großen Mengen medizinisch angewendet wird. Für solche Arzneiwirkstoffe besteht ein besonderes Interesse an einem wirtschaftlichen Herstellverfahren, das den Wirkstoff in hoher Reinheit und guten Ausbeuten liefert.

In der US-A-3 485 847 wird seine Herstellung aus Acetessigsäuremethylester, 2-Nitrobenzaldehyd und Ammoniak beschrieben. Dabei wird eine Ausbeute von ca. 72 % der Theorie erzielt.

Das nach diesem Verfahren erhaltene Nifedipin besitzt jedoch nicht die Reinheit, die bei einem Einsatz als Arzneimittelwirkstoff gefordert wird.

So beschreiben die DD 294 392 und DD 290 878 spezielle Reinigungsverfahren für (I), die einen Einsatz als Arzneimittelwirkstoff erst ermöglichen.

Solche Reinigungsoperationen sind mit Ausbeuteverlusten verbunden, so daß die Ausbeuten an reinem Nifedipin wesentlich unter denen der Syntheseverfahren liegen.

Die Erfinder der DE-A-33 12 216 (EP-A-0 124 742) stellten sich angesichts der Nachteile des Verfahrens der US-A-3 485 847 die Aufgabe, ein verbessertes Verfahren zur Herstellung der 1.4-Dihydropyridine bereitzustellen, bei dem diese in höherer Reinheit anfallen. Dazu offenbart die DE-A-33 12 216 ein Verfahren zur Herstellung von Nifedipin durch Umsetzung von 2-(2-Nitrobenzyliden)acetessigsäuremethylester und 3-Aminocrotonsäuremethylester, bei dem der Ausgangsstoff 2-(2-Nitrobenzyliden)acetessigsäuremethylester nach einem bestimmten katalytischen Verfahren hergestellt wird. Die Umsetzung des 2-(2-Nitrobenzyliden)acetessigsäuremethylesters mit dem 3-Aminocrotonsäuremethylester wird im Beispiel in Methanol unter Rückfluß (ca. 65°C) durchgeführt. Zum Erreichen einer hohen Ausbeute benötigt man nach diesem Verfahren sehr lange Reaktionszeiten. So werden die Benzylidenverbindung und der Aminocrotonsäureester 36 Stunden unter Rückfluß erhitzt. Dabei werden Ausbeuten von ca. 80 bis 87 % der Theorie erhalten. Das nach der DE-A-33 12 216 erhaltene (I) zeigt jedoch ebenfalls noch nicht die Reinheit, die bei einem Einsatz als Arzneiwirkstoff gefordert wird. So läßt sich durch HPLC-Analytik die Anwesenheit des Valenzisomeren (II) im Prozentbereich nachweisen, dessen Entfernung/Umlagerung eine zusätzliche Reinkristallisation erforderlich macht. Die Ausbeute beträgt dann nur noch 70-75 % der Theorie.

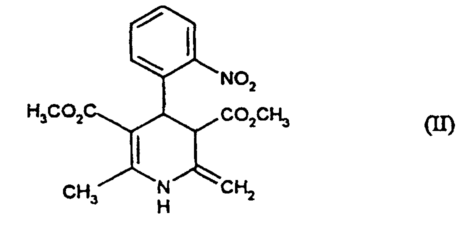

Der DE-A-44 23 445 lag die Aufgabe zugrunde, die oben beschriebenen Nachteile der DE-A-33 12 216 (EP-A-0 124 742) zu überkommen. Die DE-A-44 23 445 beschreibt ein Verfahren zur Herstellung von Nifedipin durch Umsetzung von 2-(2-Ntrobenzyliden)acetessigsäuremethylester und 3-Aminocrotonsäuremethylester in Methanol als Lösungsmittel bei Temperaturen zwischen 40 und 100°C, dadurch gekennzeichnet, daß man die Reaktion in Gegenwart von katalytischen Mengen einer niedrigen Carbonsäure durchführt. In den Beispielen der DE-A-44 23 445 wird die Reaktion in siedendem Methanol (64,5°C) und die Isolierung des Nifedipins bei etwa 0°C durchgeführt.

Das Verfahren der DE 44 23 445 führt durch den Einsatz des Katalysators zwar zu einer Verkürzung der Reaktionszeiten, die erhaltenen Produkte zeigen jedoch ebenfalls eine für den Einsatz als Arzneiwirkstoff unzureichende Reinheit. So treten in den erhaltenen Produkten neben dem oben erwähnten Valenzisomeren (II) Pyrimidin-Derivate und Lactone auf, die eine Reinkristallisation erfordern, die zu einer Gesamtausbeute von unter ca. 80 % der Theorie führt.

Die vorteilhafte Durchführung der Hantzsch-Synthese zur Herstellung von Dihydropyridinen, speziell Nifedipin in einem Autoklav oder bei erhöhter Temperatur ist in den folgenden Dokumenten beschrieben:
ES 8 600 745 beschreibt die Herstellung von Nifedipin in einem Autoklav in Ethanol als Lösungsmittel bei 110°C.
Watanabe et. al. beschreiben in Synthesis, 1983, Seite 761 die vorteilhafte Durchführung der Hantzsch-Synthese in einem Autoklav bei 110°C.
RU2057122 beschreibt die Herstellung von Nifedipin in Methanol oder i-Propanol bei Temperaturen von 76-81°C sowie die Isolierung und Reinigung des Produktes unter IR-Strahlung.

Die Erfinder unternahmen daher intensive Untersuchungen mit dem Ziel, ein Verfahren zur Herstellung von Nifedepin in hoher Reinheit und mit hohen Ausbeuten bei gleichzeitig kurzer Reaktiondauer bereitzustellen, das es erlaubt, das anfallende Nifedipin ohne weitere Reinigungsoperationen sofort als Wirkstoff in Arzneimitteln zu verwenden. Dabei fanden sie heraus, daß der im Verfahren der DE 44 23 445 verwendete Katalysator neben der gewünschten Beschleunigung der Reaktion zum Nifedipin auch zur vermehrten Bildung von Nebenprodukten führt. Außerdem fanden sie überraschend, daß bei der Durchführung der Reaktion in Abwesenheit eines Katalysators bei Temperaturen oberhalb des Siedepunktes des Methanols. also bei erhöhtem Druck, die Bildung des gewünschten Produktes, Nifedipin, beschleunigt wird, während jedoch die Bildung der Nebenprodukte nicht beschleunigt wird, und das Nifedipin nach kurzer Reaktionsdauer in so reiner Form anfällt, daß es ohne weitere Aufreinigung isoliert und als Wirkstoff in Arzneimittelzubereitungen verwendet werden kann. Auf der Grundlage dieser Befunde wurde die vorliegende Erfindung vervollständigt.

Die vorliegende Erfindung stellt ein Verfahren zur Herstellung von Nifedipin bereit, das die Reaktion von 2-(2-Nitrobenzyliden)acetessigsäuremethyl-ester und 3-Amino-crotonsäuremethylester in Methanol als Lösungsmittel umfaßt, und dadurch gekennzeichnet ist, daß man die Reaktion bei einer Temperatur zwischen 70 und 110°C unter Druck durchfuhrt. Die Durchführung der Reaktion unter Druck meint einen Druck, der oberhalb des Atmosphärendrucks, also oberhalb etwa 1 bar liegt.

Das Verfahren zur Herstellung von Nifedipin der Erfindung wird in Abwesenheit irgendeines Katalysators durchführt.

Bevorzugt wird die Reaktion in Methanol bei Temperaturen zwischen 75 und 95°C, besonders bevorzugt bei etwa 85°C durchführt. Da der Siedepunkt des Lösungsmittels (Methanol) bei Normaldruck bei etwa 65°C liegt, muß die Reaktion bei erhöhtem Druck oberhalb des Atmosphärendrucks durchgeführt werden. Dabei entspricht der Temperaturbereich von 70 bis 100°C etwa einem Druckbereich von etwa 1 bar bis etwa 3 bar.

Das Verfahren zur Herstellung von Nifedipin der Erfindung wird so durchgeführt, daß man den 2-(2-Nitrobenzyliden)acetessigsäuremethylester und den 3-Aminocrotonsäuremethylester in einem molaren Verhältnis von 2-(2-Nitrobenzyliden)acetessigsäuremethylester/3-Aminocrotonsäuremethylester von 1 bis 1,5 umsetzt.

Das Verfahren zur Herstellung von Nifedipin der Erfindung wird bevorzugt so durchgeführt, daß die Reaktionsdauer der Umsetzung des 2-(2-Nitrobenzyliden)acetessigsäuremethylesters und des 3-Amino-crotonsäuremethylesters zwischen 2 und 20 Stunden beträgt.

Das bei der Reaktion entstandene Nifedipin wird bei einer Temperatur isoliert, die zwischen 30°C und 50°C, bevorzugt zwischen 35°C und 45°C und besonders bevorzugt bei etwa 40°C liegt.

Das Reaktionsgemisch der Erfindung bildet oberhalb 90°C eine klare Lösung, so daß unter diesen Bedingungen eine bei der Synthese von Arzneiwirkstoffen übliche Filtration durchgeführt werden kann, was einen weiteren Vorteil des Arbeitens im angegeben Temperaturbereich bei erhöhtem Druck darstellt

Das Verfahren der Erfindung führt im angegebenen Temperaturfenster unter erhöhtem Druck unerwartet zu einer signifikanten Reduzierung der Reaktionszeit. Bereitsnach 8 bis 12, bevorzugt 8 bis 10 Stunden ist die Reaktion beendet. Es war dabei völlig überraschend, daß dabei auch das Valenzisomere (II), das bei den im Stand der Technik beschriebenen Verfahren als Neben-produkt auftritt, nicht mehr detektiert werden kann.

Weiterhin wurde überraschend gefunden, daß nach Isolierung des kristallinen (I) aus der Suspension bei Temperaturen oberhalb von 25°C das Nifedipin in einer Reinheit anfällt, die den Anforderungen, die an einen Arzneimittelwirkstoff gestellt werden, voll entspricht. Der Wegfall einer Reinigungsoperation bei der Herstellung des Nifedipins gemäß dem Verfahren der Erfindung stellt gegenüber den im Stand der Technik beschriebenen Verfahren einen bedeutenden wirtschaftlichen Faktor dar. Dabei ist insbesondere zu berücksichtigen, daß das Produkt der DE-A-44 23 445 nach der erforderlichen Reinigungsoperation in einer geringeren Gesamtausbeute anfällt, so daß das Verfahren der Erfindung sowohl durch den Wegfall der Reinigungsoperation als auch die höhere Gesamtausbeute wirtschaftlicher ist.

Nach dem Verfahren der Erfindung wird Nifedipin mit einer Ausbeute von 85 % der Theorie in einer Qualität erhalten, die nach den bisher bekannten Syntheseverfahren nur durch eine zusätzliche Reinkristallisation mit den dabei auftretenden Ausbeuteverlusten erhalten werden kann.

### Ansführungsbeispiel

1185 g (4,76 Mol) 2-(2-Nitrobenzyliden)acetessigsäuremethylester werden zusammen mit 600 g (5,22 Mol) 3-Aminocrotonsäuremethylester in 1900 ml Methanol in einem verschlossenen Rührwerkskessel auf 85°C erwärmt und 10 Std. bei 85°C gerührt. Danach wird auf 40°C abgekühlt. Das Kristallisat wird abgesaugt und mit Methanol und Wasser gewaschen. Nach dem Trocknen im Vakuum erhält man 1400 g (= 85 % d. Th.) Nifedipin.

## Patentansprüche

1. Verfahren zur Herstellung von Nifedipin, das die Reaktion von 2-(2-Nitrobenzyliden)acetessigsäuremethylester und 3-Aminocrotonsäuremethylester in Methanol als Lösungsmittel einem molaren Verhältnis 2-(2-Nitrobenzyliden)acetessigsäuremethylester/3-Aminocrotonsäuremethylester von 1 bis 1,5 umfaßt, **dadurch gekennzeichnet, daß** man die Reaktion bei einer Temperatur zwischen 70 und 110°C unter Druck in Abwesenheit eines Katalysators durchführt und das bei der Reaktion entstandene Nifedipin bei einer Temperatur isoliert, die zwischen 30°C und 50°C liegt.

2. Verfahren zur Herstellung von Nifedipin nach Anspruch 1, **dadurch gekennzeichnet, daß** man das bei der Reaktion entstandene Nifedipin bei einer Temperatur isoliert, die zwischen 35°C und 45°C liegt.

3. Verfahren zur Herstellung von Nifedipin nach Anspruch 1, **dadurch gekennzeichnet, daß** man das bei der Reaktion entstandene Nifedipin bei einer Temperatur isoliert, die bei etwa 45°C liegt.

4. Verfahren zur Herstellung von Nifedipin nach einem der Ansprüche 1 bis 3, **dadurch gekennzeichnet, daß** man die Reaktion bei Temperaturen zwischen 75 und 95°C durchführt.

5. Verfahren zur Herstellung von Nifedipin nach einem der Ansprüche 1 bis 4, **dadurch gekennzeichnet, daß** die Reaktionsdauer der Umsetzung des 2-(2-Nitrobenzyliden)acetessigsäuremethylesters und des 3-Aminocrotonsäuremethylesters zwischen 2 und 20 Stunden beträgt.

## Claims

1. Process for the preparation of nifedipine, which comprises the reaction of methyl 2-(2-nitrobenzylidene)acetoacetate and methyl 3-aminocrotonate in methanol as solvent in a molar ratio of methyl 2-(2-nitrobenzylidene)acetoacetate/methyl 3-aminocrotonate of 1 to 1.5, **characterized in that** the reaction is carried out under pressure at a temperature between 70 and 110°C in the absence of a catalyst and the nifedipine formed in the reaction is isolated at a temperature which is between 30°C and 50°C.

2. Process for the preparation of nifedipine according to Claim 1, **characterized in that** the nifedipine formed in the reaction is isolated at a temperature which is between 35°C and 45°C.

3. Process for the preparation of nifedipine according to Claim 1, **characterized in that** the nifedipine formed in the reaction is isolated at a temperature which is approximately 45°C.

4. Process for the preparation of nifedipine according to any of Claims 1 to 3, **characterized in that** the reaction is carried out at temperatures between 75 and 95°C.

5. Process for the preparation of nifedipine according to any of Claims 1 to 4, **characterized in that** the reaction time of the reaction of the methyl 2-(2-nitrobenzylidene)acetoacetate and of the methyl 3-aminocrotonate is between 2 and 20 hours.

## Revendications

1. Procédé de production de nifédipine, qui comprend la réaction de l'ester méthylique d'acide 2-(2-nitrobenzylidène)acétylacétique et de l'ester méthylique d'acide 3-aminocrotonique dans du méthanol utilisé comme solvant dans un rapport molaire ester méthylique d'acide 2-(2-nitrobenzylidène)acétylacétique/ester méthylique d'acide 3-aminocrotonique de 1 à 1,5, **caractérisé en ce qu'**on conduit la réaction à une température comprise entre 70 et 110°C sous pression en l'absence d'un catalyseur, et on isole la nifédipine produite dans la réaction à une température qui se situe entre 30°C et 50°C.

2. Procédé de production de nifédipine suivant la revendication 1, **caractérisé en ce qu'**on isole la nifédipine produite dans la réaction à une température qui se situe entre 35°C et 45°C.

3. Procédé de production de nifédipine suivant la revendication 1, **caractérisé en ce qu'**on isole la nifédipine produite dans la réaction à une température qui se situe à environ 45°C.

4. Procédé de production de nifédipine suivant l'une des revendications 1 à 3, **caractérisé en ce qu'**on conduit la réaction à des températures comprises entre 75 et 95°C.

5. Procédé de production de nifédipine suivant l'une des revendications 1 à 4, **caractérisé en ce que** la réaction entre l'ester méthylique d'acide 2-(2-nitrobenzylidène)acétylacétique et l'ester méthylique d'acide 3-aminocrotonique a une durée comprise entre 2 et 20 heures.
